# EUROPEAN PATENT APPLICATION

(11) **EP 4 740 899 A1**
(43) Date of publication of application: **13.05.2026**
(21) Application number: 24836373.1
(22) Date of filing: 05.07.2024
(51) Int. Cl.: A61C 9/00, A61C 19/04, A61B 1/24, A61B 5/00, G06T 1/00, G06F 30/00, G06T 7/33, G06T 11/00, G06T 19/20

(54) **METHOD, APPARATUS, AND RECORDING MEDIUM RECORDING INSTRUCTIONS FOR ALIGNING 3D DATA OF ORAL CAVITY WITH OCCLUSAL PLANE**

(30) Priority: 06.07.2023 KR 20230087632; 04.07.2024 KR 20240088434
(71) Applicant: Medit Corp., Seoul 07207 (KR)
(72) Inventor: LEE, Sunghoon, Seoul 07207 (KR)
(74) Representative: MFG Patentanwälte Meyer-Wildhagen Meggle-Freund Gerhard PartG mbB
(86) International application number: PCT/KR2024/009554
(87) International publication number: WO 2025/009926

(57) **Abstract**

Disclosed is a technique for aligning three-dimensional data of an oral cavity to an occlusal plane. A method performed by an electronic device according to an aspect of the present disclosure may include the steps of: obtaining first data which is three-dimensional data for an oral cavity including a plurality of teeth; determining three-dimensional center coordinates of each of the plurality of teeth indicated by the first data; determining one or more target teeth among the plurality of teeth; determining one or more reference center points respectively corresponding to the one or more target teeth on an occlusal plane for aligning the plurality of teeth; and generating a transformation matrix for aligning the first data to the occlusal plane on the basis of three-dimensional center coordinates of each of the one or more target teeth and the one or more reference center points.

## Description

### TECHNICAL FIELD

The present disclosure relates to a technology for aligning three-dimensional data of an oral cavity with an occlusal plane.

### BACKGROUND

The manufacture of dental prostheses and oral appliances requires accurate oral structure data of a patient. In the past, a scheme of making a model by taking an impression of an oral condition of patient by using a physical impression material was mainly used, but with development of technology, a scheme of obtaining a three-dimensional image of an internal structure of the oral cavity of the patient by using a three-dimensional oral scanner is being used. A three-dimensional scanner may project lasers and light rays from various angles, and measure the degree of reflection of the corresponding light ray so as to convert an accurate shape of an internal oral structure including teeth and gingiva into three-dimensional data.

Based on three-dimensional data, prostheses, crowns, bridges, implants, orthodontic appliances, and the like can be designed and manufactured according to the oral condition of an individual patient by using a Computer-Aided Design and Computer-Aided Manufacturing (CAD/CAM) system. When various processing based on three-dimensional data is performed to design an oral appliance, the three-dimensional data needs to be precisely aligned with an occlusal plane so that each processing procedure can be performed as intended.

### DISCLOSURE

### TECHNICAL PROBLEM

A technical problem to be solved through at least one embodiment of the present disclosure provides a technology enabling three-dimensional data of an oral cavity to be precisely aligned with an occlusal plane.

A technical problem to be solved through at least one embodiment of the present disclosure provides a technology enabling a transformation matrix for aligning three-dimensional data with an occlusal plane to be generated.

A technical problem to be solved through at least one embodiment of the present disclosure provides a technology enabling three-dimensional data to be precisely aligned with an occlusal plane even though tooth pairs corresponding each other do not exist in the three-dimensional data.

The technical problems of the present disclosure are not limited to the above-mentioned technical problems, and other unmentioned technical problems can be clearly understood by those skilled in the art of the present disclosure from the description below.

### TECHNICAL SOLUTION

A method performed by an electronic device according to one aspect of the present disclosure may include: obtaining first data corresponding to three-dimensional data for an oral cavity comprising a plurality of teeth; determining three-dimensional center coordinates of each of the plurality of teeth indicated by the first data; determining one or more target teeth among the plurality of teeth; determining one or more reference center points corresponding to the one or more target teeth, respectively, on an occlusal plane for aligning the plurality of teeth; and generating a transformation matrix for aligning the first data to the occlusal plane, based on the three-dimensional center coordinates of each of the one or more target teeth and the one or more reference center points.

According to one embodiment of the present disclosure, wherein the determining of the one or more target teeth among the plurality of teeth comprises: classifying each of the plurality of teeth as one of a first tooth group and a second tooth group; determining whether there is a first pair of teeth at positions corresponding to each other with reference to a midline of the oral cavity and belonging to the first tooth group; and determining whether there is a second pair of teeth at positions corresponding to each other with reference to the midline and belonging to the second tooth group.

According to one embodiment of the present disclosure, the method further comprising determining an identification number of each of the plurality of teeth, wherein the determining of whether there is the first pair of teeth and the determining of whether there is the second pair of teeth are based on the identification number of each of the plurality of teeth.

According to one embodiment of the present disclosure, wherein the determining of the one or more target teeth among the plurality of teeth comprises, in response to determination that both the first pair of teeth and the second pair of teeth exist, determining four teeth included in the first pair of teeth and the second pair of teeth as the one or more target teeth.

According to one embodiment of the present disclosure, wherein the determining of the one or more target teeth among the plurality of teeth further comprises when there are a plurality of pairs of teeth at positions corresponding to each other with reference to the midline among one or more teeth included in the first tooth group, determining, as the first pair of teeth, a pair of teeth comprising a tooth closest from the midline among the plurality of teeth at positions corresponding to each other with reference to the midline.

According to one embodiment of the present disclosure, wherein the determining of the one or more target teeth among the plurality of teeth further comprises when there are a plurality of pairs of teeth at positions corresponding to each other with reference to the midline among one or more teeth included in the second tooth group, determining, as the second pair of teeth, a pair of teeth comprising a tooth closest from the midline among the plurality of teeth at positions corresponding to each other with reference to the midline.

According to one embodiment of the present disclosure, wherein the generating of the transformation matrix comprises, in response to determination that both the first pair of teeth and the second pair of teeth exist, generating the transformation matrix so that two or more straight lines derived based on three-dimensional center coordinates of the four teeth align with corresponding straight lines on the occlusal plane.

According to one embodiment of the present disclosure, wherein the determining of the one or more target teeth among the plurality of teeth comprises, in response to determination that at least one of the first pair of teeth and the second pair of teeth does not exist, determining all of the plurality of teeth as the one or more target teeth.

According to one embodiment of the present disclosure, wherein the generating of the transformation matrix comprises, in response to determination that at least one of the first pair of teeth and the second pair of teeth does not exist, generating the transformation matrix so that three-dimensional center coordinates of each of the one or more target teeth are moved as close as possible to a corresponding reference center point of the occlusal plane and an oral cavity movement amount indicated by the first data is minimized.

According to one embodiment of the present disclosure, wherein the determining of the three-dimensional center coordinates of each of the plurality of teeth indicated by the first data comprises: generating, based on the first data, second data corresponding to two-dimensional data for the oral cavity; identifying each of the plurality of teeth from the second data; determining two-dimensional center coordinates of each of the plurality of teeth; and determining three-dimensional center coordinates of each of the plurality of teeth, based on the two-dimensional center coordinates.

According to one embodiment of the present disclosure, wherein the generating of the second data corresponding to the two-dimensional data for the oral cavity comprises: identifying a tooth area indicated by the first data, wherein the tooth area comprises the plurality of teeth; adding a color indicating a curvature to each point of the tooth area; and generating the second data, based on data of the tooth area in which the color is added, among the first data.

According to one embodiment of the present disclosure, wherein the determining of the two-dimensional center coordinates of each of the plurality of teeth comprises: identifying each of the plurality of teeth indicated by the second data; generating a plurality of bounding boxes corresponding to the plurality of teeth, respectively, wherein the plurality of boxes have a smallest quadrilateral shape comprising an area in which each of the plurality of teeth is positioned in the second data; and determining a center of each of the plurality of bounding boxes as two-dimensional center coordinates of a corresponding tooth.

According to one embodiment of the present disclosure, wherein the determining of the two-dimensional center coordinates of each of the plurality of teeth comprises applying the second data to a training model to obtain output data, wherein the output data identifies each of the plurality of teeth and indicates the two-dimensional center coordinates of each of the plurality of teeth.

According to one embodiment of the present disclosure, wherein the determining of the three-dimensional center coordinates of each of the plurality of teeth comprises: determining a plurality of first points which correspond to the two-dimensional center coordinates of each of the plurality of teeth and are on a plane identical or parallel to the occlusal plane; determining a plurality of second points on surfaces of the plurality of teeth, which correspond to the plurality of first points, wherein each of the plurality of second points is an intersection point between a straight line which is parallel to a normal line of the occlusal plane and passes through a corresponding first point and a surface of each of the plurality of teeth; and determining the plurality of second points as the three-dimensional center coordinates of each of the plurality of teeth.

According to one embodiment of the present disclosure, wherein the determining of the three-dimensional center coordinates of each of the plurality of teeth comprises: determining, based on the plurality of second points, a plurality of third points corresponding to cusp points of each of the plurality of teeth; and determining the plurality of third points as the three-dimensional center coordinates of each of the plurality of teeth.

According to one embodiment of the present disclosure, wherein the determining of the third point of each of the plurality of teeth comprises determining, as the third point of each of the plurality of teeth, a point at which a curvature has a maximum value among points obtained by moving the plurality of second points in a buccal direction and a lingual direction along the surface of each of the plurality of teeth.

According to one embodiment of the present disclosure, the method further comprising aligning the first data to the occlusal plane, based on the transformation matrix.

According to one embodiment of the present disclosure, the method further comprising: segmenting surfaces of a plurality of teeth indicated by the first data into one or more polygons; determining one or more first normal vectors for each of the one or more polygons; determining a second normal vector corresponding to the first data, based on the one or more first normal vectors; determining a third normal vector corresponding to a normal vector of the occlusal plane; and generating a rotation matrix for rotating the first data so that the second normal vector aligns with the third normal vector.

An electronic device according to one aspect of the present disclosure may include: one or more processors; and one or more memories in which instructions executed by the one or more processes are stored, wherein the one or more processors are configured to, when the instructions are executed by the one or more processors, execute the method according to the present disclosure.

A non-transitory computer-readable recording medium recording instructions according to one aspect of the present disclosure which, when executed by one or more processors, cause the one or more processors to perform operations, wherein the instructions are configured to cause the one or more processors to execute the method according to the present disclosure.

### ADVANTAGEOUS EFFECTS

According to various embodiments of the present disclosure, three-dimensional data of an oral cavity can be precisely aligned with an occlusal plane.

According to various embodiments of the present disclosure, a transformation matrix for aligning three-dimensional data with an occlusal plane can be generated.

According to various embodiments of the present disclosure, three-dimensional data can be precisely aligned with an occlusal plane even though tooth pairs corresponding each other do not exist in the three-dimensional data.

The advantageous effects according to technical idea of the present disclosure are not limited to the above-described advantageous effects, and other unmentioned advantageous effects can be clearly understood by those skilled in the art of the present disclosure from the description below.

### DESCRIPTION OF DRAWINGS

FIG. 1 illustrates obtaining an image of an oral cavity of a patient by using an oral scanner according to an embodiment of the present disclosure.
FIG. 2A is a block diagram illustrating an electronic device and an oral scanner according to an embodiment of the present disclosure. FIG. 2B is a perspective view of an oral scanner according to an embodiment of the present disclosure.
FIG. 3 illustrates a method of generating a three-dimensional image of an oral cavity according to an embodiment of the present disclosure.
FIG. 4 illustrates aligning first data to an occlusal plane according to an embodiment of the present disclosure.
FIG. 5A illustrates obtained first data according to an embodiment of the present disclosure.
FIG. 5B illustrates first data rotated based on a normal vector of an occlusal plane according to an embodiment of the present disclosure.
FIGS. 6A and 6B illustrate second data according to an embodiment of the present disclosure.
FIGS. 7A and 7B illustrate a result of inference of a training model for second data according to an embodiment of the present disclosure.
FIG. 8 illustrates an example of an inference result of a training model according to an embodiment of the present disclosure.
FIG. 9 illustrates a process of determining three-dimensional center coordinates of each of a plurality of teeth by using a ray projection according to an embodiment of the present disclosure.
FIG. 10A illustrates reference center points on an occlusal plane according to an embodiment of the present disclosure.
FIGS. 10B and 10C illustrate a process of aligning first data to an occlusal plane according to an embodiment of the present disclosure.
FIG. 11A illustrates full arch data before alignment to an occlusal plane according to an embodiment of the present disclosure.
FIG. 11B illustrates full arch data obtained after alignment to an occlusal plane according to an embodiment of the present disclosure.
FIG. 12A illustrates partial arch data before alignment to an occlusal plane according to an embodiment of the present disclosure.
FIG. 12B illustrates partial arch data obtained after alignment to an occlusal plane according to an embodiment of the present disclosure.
FIG. 13 is a flowchart illustrating a method of aligning three-dimensional data of an oral cavity to an occlusal plane according to an embodiment of the present disclosure.

### DETAILED DESCRIPTION

Various embodiments described herein are illustrated for the purpose of clarifying the technical idea of the present disclosure, and are not intended to limit the present disclosure to any specific embodiment. The technical idea of the present disclosure includes various modifications, equivalents, alternatives and embodiments selectively combined from all or part of individual embodiments described herein. Furthermore, the scope of the technical idea of the present disclosure is not limited to the various embodiments described below, and the detailed description thereof.

In the present disclosure, terms including technical or scientific terms, may have meanings that are generally understood by those ordinarily skilled in the art to which this disclosure belongs, unless otherwise defined.

In the present disclosure, the expressions "include," "may include," "provided with," "may be provided with," "have," "may have," and the like, mean that corresponding features (e.g., functions, operations, or components, etc.) are present, but do not exclude the presence of other additional features. That is, such expressions should be understood as open-ended terms that include the possibility of including other embodiments.

In the present disclosure, the singular of an expression may include the meaning of the plural of the expression unless otherwise indicated in the context clearly dictates otherwise, and the same applies to singular forms of expressions as set forth in the claims.

In the present disclosure, the expressions "1^{st}," "2^{nd}," "first," "second" and the like are used to distinguish one object from another in referring to plural same objects unless otherwise indicated in the context, and do not limit the order or importance of the objects.

In the present disclosure, the expressions "A, B and C," "A, B or C," "A, B and/or C," "at least one of A, B, and C," "at least one of A, B, or C," "at least one of A, B, and/or C," and the like may be used to refer to each listed item or any possible combination of the listed items may be provided. For example, the expression "at least one of A and B" may be used to refer to all of (1) at least one A, (2) at least one B, and (3) at least A and at least B.

In the present disclosure, the expression "based on ..." is used to describe one or more factors that affect the action or operation of a decision or determination, described in a phrase or sentence in which the expression is contained, and does not exclude additional factors that influence the action or operation of the corresponding decisions or determination.

In the present disclosure, the expression that information C is determined based on information A and information B may mean that information A and information B are considered in determining information C, and does not exclude that information other than information A and information B is additionally considered.

In the present disclosure, the expression that a component (e.g., a first component) is "connected" or "coupled" to another component (e.g., a second component) may mean that the one component is connected or coupled to the other component not only directly, but also via another new component (e.g., a third component).

In the present disclosure, the expression "configured to ..." is intended to encompass, depending on the context, the meanings of "set to ...," "having performance of ...," "altered to ...," "made to ...," and "enabled to ...," and the like. The corresponding expression is not limited to the meaning "specifically designed in hardware," and for example, a processor configured to perform a specific operation may mean a generic-purpose processor that can perform the specific operation by executing software.

In the present disclosure, a training model may be designed to implement a human brain structure on a computer and may include a plurality of network nodes that mimic neurons in a human neural network and have weights. The plurality of network nodes may have connections to one another by mimicking the synaptic activity of neurons transmitting and receiving signals through synapses. In a training model, the plurality of network nodes may be located on layers having different depths to transmit and receive data based on convolutional connections. The training model may be, for example, an artificial neural network, a time-series analysis model, etc. Here, the time-series analysis model is a model for analyzing time-series data, and may include a recurrent neural network (RNN) model, a long short-term memory (LSTM) model, and an autoregressive integrated moving average (ARIMA) model. The present disclosure is not limited thereto, and various types of models for analyzing data can be used.

In the present disclosure, a "training process" may mean a process in which a training model repeats a process of extracting and analyzing features (patterns) of a pair of input data and output data of training data and derive a correlation between the input and output data, so that a parameter of the training model can be optimized based on the correlation between the input and output data.

In the present disclosure, an "inference" process may mean a process in which a training model applies a pre-learned pattern to new input data to generate output data as a result of prediction or classification of the input data.

In the present disclosure, directional terms such as "superior" and "upper" are based on the orientation of the occlusal plane relative to the teeth as shown in the accompanying drawings unless otherwise specifically defined in the corresponding description, and directional terms such as "inferior" and "lower" mean the opposite direction. A plurality of teeth included in the oral cavity shown in the accompanying drawings may be included in the maxillary arch or the mandibular arch, but the directional terms may be understood according thereto.

Hereinafter, embodiments of the present disclosure will be described with reference to the accompanying drawings. In the accompanying drawings and the descriptions of the drawings, substantially equivalent elements may be given the same reference numerals. In addition, in the following description of the various embodiments, a redundant description of the same or corresponding components may be omitted, but this does not mean that the components are not included in the embodiment.

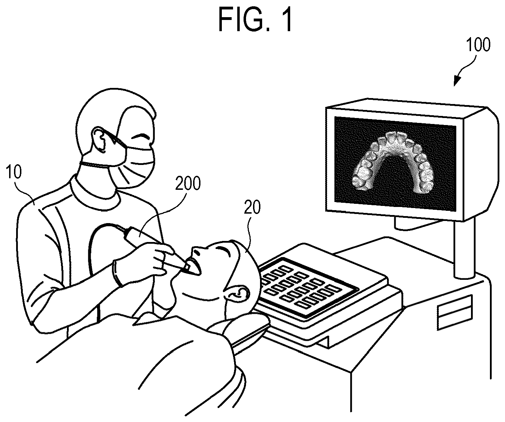

FIG. 1 illustrates obtaining an image of an oral cavity of a patient by using a three-dimensional scanner 200 according to an embodiment of the present disclosure. According to an embodiment, the three-dimensional scanner 200 may be a dental medical device for obtaining an image of the inside of an oral cavity of a subject 20. For example, the three-dimensional scanner 200 may be an intraoral scanner. As illustrated in FIG. 1, a user 10 (e.g., a dentist or a dental hygienist) may obtain an image of an oral cavity of the subject 20 from the subject 20 (e.g., a patient) by using the three-dimensional scanner 200. In another example, the user 10 may also obtain an image of the oral cavity of the subject 20 from a diagnostic model (e.g., a plaster model or an impression model) obtained by taking an impression of the shape of the oral cavity of the subject 20. Hereinafter, for convenience of description, it is described that an image of the oral cavity of the subject 20 is obtained by scanning the oral cavity of the subject 20, but the present disclosure is not limited thereto, and it is also possible to obtain an image of another part (e.g., an ear of the subject 20) of the subject 20. The three-dimensional scanner 200 may have a shape enabling insertion into and withdrawal from the oral cavity, and may be a handheld scanner enabling the user 10 to freely adjust a scan distance and a scan angle.

The three-dimensional scanner 200 according to an embodiment may be inserted into the oral cavity of the subject 20 and may scan the inside of the oral cavity in a contactless manner, so as to obtain an image of the oral cavity. The image of the oral cavity may include at least one tooth or gingiva. The three-dimensional scanner 200 may emit light to the oral cavity of the subject 20 (e.g., the at least one tooth or gingiva of the subject 20) by using a light source (or a projector), and receive light reflected from the oral cavity of the subject 20 through a camera (or at least one image sensor). According to another embodiment, the three-dimensional scanner 200 may scan a diagnostic model of the oral cavity to obtain an image of the diagnostic model of the oral cavity. When the diagnostic model of the oral cavity is a diagnostic model obtained by taking an impression of the shape of the oral cavity of the subject 20, the image of the diagnostic model of the oral cavity may be an image of the oral cavity of the subject. Hereinafter, for convenience of description, a case where an image of an oral cavity is obtained by scanning the inside of the oral cavity of the subject 20 is assumed and described, but the present disclosure is not limited thereto.

The three-dimensional scanner 200 according to an embodiment may obtain, based on information received through a camera, a surface image of the oral cavity of the subject 20 as a two-dimensional image. The surface image of the oral cavity of the subject 20 may include at least one of the at least one tooth or gingiva of the subject 20, an artificial structure, and the cheek, tongue, or lips of the subject 20. The surface image of the oral cavity of the subject 20 may be a two-dimensional image.

The two-dimensional image of the oral cavity, obtained by the three-dimensional scanner 200 according to an embodiment, may be transmitted to a wiredly or wirelessly connected electronic device 100. The electronic device 100 may be a computer device or a portable communication device. The electronic device 100 may generate, based on the two-dimensional image of the oral cavity, received from the three-dimensional scanner 200, a three-dimensional image of the oral cavity (or referred to as a three-dimensional oral image, a three-dimensional oral model, a three-dimensional scan model, a tooth model, a three-dimensional oral representation, a three-dimensional numerical entity, etc.), which indicates the oral cavity in three-dimensions. Hereinafter, the three-dimensional image may be exchangeable used with first data. The electronic device 100 may generate, based on the received two-dimensional image of the oral cavity, the three-dimensional image of the oral cavity by modeling the internal structure of the oral cavity in three-dimensions.

The three-dimensional image may include not only geometry information in the three-dimensional space of the subject but also information such as the color, texture, and material. The format of the three-dimensional image may be one of, for example, a standard triangle language (STL), an OBJ, and a polygon file format, but the present disclosure is not limited thereto. The "polygon" may mean a polygon corresponding to the smallest unit used to represent a stereoscopic image of a three-dimensional oral model. For example, the surface of the three-dimensional image may be represented by triangular or quadrilateral polygons. For example, the polygon may include at least three vertices and one face. The three-dimensional image may include information on the location, color, and normal line on the three-dimensional space for each vertex. A mesh is composed of a plurality of polygons, and may indicate the geometry of the subject on the three-dimensional space. The larger the number of polygons representing the three-dimensional image, the more the subject can be represented in detail.

The three-dimensional scanner 200 according to another embodiment may scan the oral cavity of the subject 20 to obtain a two-dimensional image of the oral cavity, may generate, based on the obtained two-dimensional image of the oral cavity, a three-dimensional image of the oral cavity, and may transmit the generated three-dimensional image of the oral cavity to the electronic device 100.

The electronic device 100 according to an embodiment may be communicatively connected to a cloud server (not shown). In the case above, the electronic device 100 may transmit the two-dimensional image of the oral cavity or the three-dimensional image of the oral cavity of the subject 20 to the cloud server, and the cloud server may store the two-dimensional image of the oral cavity or the three-dimensional image of the oral cavity of the subject 20, which is received from the electronic device 100.

According to another embodiment, for the three-dimensional scanner, a table scanner (not shown) used while being fixed at a specific position may be used, in addition to a handheld scanner used while being inserted into the oral cavity of the subject 20. The table scanner may generate a three-dimensional image of a diagnostic model of the oral cavity by scanner in the diagnostic model of the oral cavity. In the case above, a light source (or project) and a camera of the table scanner are fixed, and thus a user may scan the diagnostic model of the oral cavity while moving an arm for fixing the diagnostic model of the oral cavity. There is a relatively low possibility for the table scanner to cause noise due to the intervention of another object between the camera and the diagnostic model during the scanning operation, compared to the handheld scanner, but embodiments of the present disclosure are applicable to the table scanner and other three-dimensional scanners as well as the handheld scanner.

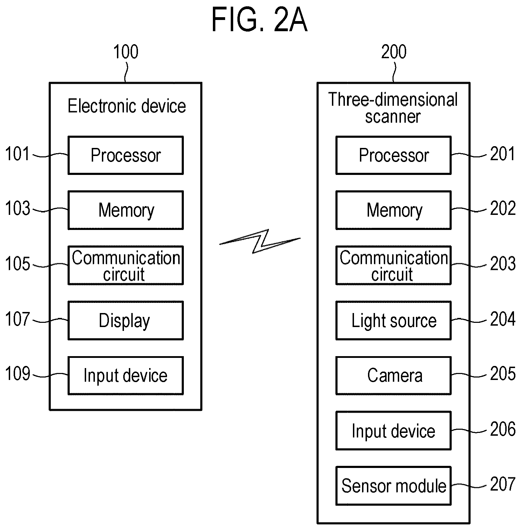

FIG. 2A is a block diagram illustrating an electronic device 100 and a three-dimensional scanner 200 according to an embodiment of the present disclosure. The electronic device 100 and the three-dimensional scanner 200 may be communicatively connected to each other through a wired or wireless communication network, and transmit and receive various data to and from each other.

The three-dimensional scanner 200 according to an embodiment may include a processor 201, a memory 202, a communication circuit 203, a light source 204, a camera 205, an input device 206, and/or a sensor module 207. At least one of the elements included in the three-dimensional scanner 200 may be omitted, or another element may be added to the three-dimensional scanner 200. Additionally or alternatively, some elements may be implemented to be integrated, or may be implemented as a single entity or multiple entities. At least some elements in the three-dimensional scanner 200 may be connected to each other through a bus, a general purpose input/output (GPIO), a serial peripheral interface (SPI), a mobile industry processor interface (MIPI), or the like, and exchange data and/or a signal from each other.

The processor 201 of the three-dimensional scanner 200 according to an embodiment may be configured to perform computation or data processing of control and/or communication of each of the elements of the three-dimensional scanner 200, and may be operatively connected to the elements of the three-dimensional scanner 200. The processor 201 may load, to the memory 202, a command or data received from other elements of the three-dimensional scanner 200, may process the command or data stored in the memory 202, and may store result data. The memory 202 of the three-dimensional scanner 200 according to an embodiment may store instructions for the above-described operation of the processor 201.

According to an embodiment, the communication circuit 203 of the three-dimensional scanner 200 may establish a wired or wireless communication channel with an external device (e.g., the electronic device 100), and transmit or receive various data to or from the external device. According to an embodiment, the communication circuit 203 may include at least one port for connection to the external device through a wired cable, in order to wiredly communicate with the external device. In the case above, the communication circuit 203 may perform communication with the external device wiredly connected through the at least one port. According to an embodiment, the communication circuit 203 may include a cellular communication module and may be configured to be connected to a cellular network (e.g., 3G, LTE, 5G, WiBro, or WiMAX). According to an embodiment, the communication circuit 203 may include a short-distance communication module so as to transmit and receive data to or from the external device by using short-distance communication (e.g., Wi-Fi, Bluetooth^{™}, Bluetooth Low Energy (BLE), and UWB), but the present disclosure is not limited thereto. According to an embodiment, the communication circuit 203 may include a contactless communication module for contactless communication. The contactless communication may include at least one contactless short-distance communication technology such as near field communication (NFC) communication, radio frequency identification (RFID) communication, or magnetic secure transmission (MST) communication.

The light source 204 of the three-dimensional scanner 200 according to an embodiment may emit light toward the oral cavity of the subject 20. For example, the light emitted from the light source 204 may correspond to structured light having a predetermined pattern (e.g., a stripe pattern in which straight lines of different colors consecutively appear). The structed line pattern may be generated using, for example, a pattern mask or a digital micro-mirror device (DMD), but the present disclosure is not limited thereto. The camera 205 of the three-dimensional scanner 200 according to an embodiment may receive light reflected by the oral cavity of the subject 20, so as to obtain an image of the oral cavity of the subject 20. The camera 205 may include a left camera corresponding to the left-eye field of view and a right camera corresponding to the right-eye field of view in order to construct a three-dimensional image according to an optical triangulation scheme, for example. The camera 205 may include at least one image sensor such as a CCD sensor or a CMOS sensor.

The input device 206 of the three-dimensional scanner 200 according to an embodiment may receive a user input for controlling the three-dimensional scanner. The input device 206 may include a button for receiving a push operation of the user 10, a touch panel for detecting a touch of the user 10, and a speech recognition device including a microphone. For example, the user 10 may control the start or stop of scanning by using the input device 206.

The sensor module 207 of the three-dimensional scanner 200 according to an embodiment may detect an operational state or an environment state (e.g., a user's action) of the three-dimensional scanner 200, and generate an electrical signal corresponding to the detected state. The sensor module 207 may include at least one of, for example, a gyro sensor, an acceleration sensor, a gesture sensor, a proximity sensor, and an infrared sensor. The user 10 may control the start or stop of scanning by using the sensor module 207. For example, when the user 10 moves the three-dimensional scanner 200 while gripping the same, the three-dimensional scanner 200 may control the processor 201 to start a scanner operation when the angular velocity measured through the sensor module 207 exceeds a pre-configured threshold value.

According to an embodiment, the three-dimensional scanner 200 may start scanning by receiving a user input for starting scanning through the input device 206 of the three-dimensional scanner 200 or the input device 109 of the electronic device 100, or according to the processing of the processor 201 of the three-dimensional scanner 200 or the processor 101 of the electronic device 100. When the user 10 scans the inside of the oral cavity of the subject 20 through the three-dimensional scanner 200, the three-dimensional scanner 200 may generate a two-dimensional image of the oral cavity of the subject 20, and may transmit the two-dimensional image of the oral cavity of the subject 20 to the electronic device 100 in real time. The electronic device 100 may display the received two-dimensional image of the oral cavity of the subject 20 through the display 107. In addition, the electronic device 100 may generate (construct), based on the two-dimensional image of the oral cavity of the subject 20, the three-dimensional image of the oral cavity of the subject 20, and may display the three-dimensional image of the oral cavity through the display 107. The electronic device 100 may display the three-dimensional image, which is being generated, through the display 107 in real time.

The electronic device 100 according to an embodiment may include one or more processors 101, one or more memories 103, a communication circuit 105, a display 107, and/or an input device 109. At least one of the elements included in the electronic device 100 may be omitted, or another element may be added to the electronic device 100. Additionally or alternatively, some elements may be implemented to be integrated, or may be implemented as a single entity or multiple entities. At least some elements in the electronic device 100 may be connected to each other through a bus, a general purpose input/output (GPI), a serial peripheral interface (SPI), a mobile industry processor interface (MIPI), or the like, and exchange data and/or a signal from each other.

According to an embodiment, the one or more processors 101 of the electronic device 100 may be configured to perform computation or data processing of control and/or communication of each of the elements (e.g., the memory 103) of the electronic device 100. The one or more processors 101 may be operatively connected to the elements of the electronic device 100, for example. The one or more processors 101 may load, to the one or more memories 103, a command or data received from other elements of the electronic device 100, may process the command or data stored in the one or more memories 103, and may store result data.

According to an embodiment, the one or more memories 103 of the electronic device 100 may store instructions for the above-described operation of the one or more processors 101. The one or more memories 103 may store data (e.g., a two-dimensional image of the oral cavity, obtained through oral scanning) received from the three-dimensional scanner 200.

According to an embodiment, the communication circuit 105 of the electronic device 100 may establish a wired or wireless communication channel with an external device (e.g., the three-dimensional scanner 200 or a cloud server), and transmit or receive various data to or from the external device. According to an embodiment, the communication circuit 105 may include at least one port for connection to the external device through a wired cable, in order to wiredly communicate with the external device. In the case above, the communication circuit 105 may perform communication with the external device wiredly connected through the at least one port. According to an embodiment, the communication circuit 105 may include a cellular communication module and may be configured to be connected to a cellular network (e.g., 3G, LTE, 5G, WiBro, or WiMAX). According to an embodiment, the communication circuit 105 may include a short-distance communication module so as to transmit and receive data to or from the external device by using short-distance communication (e.g., Wi-Fi, BluetoothTM, Bluetooth Low Energy (BLE), and UWB), but the present disclosure is not limited thereto. According to an embodiment, the communication circuit 105 may include a contactless communication module for contactless communication. The contactless communication may include at least one contactless short-distance communication technology such as near field communication (NFC) communication, radio frequency identification (RFID) communication, or magnetic secure transmission (MST) communication.

The display 107 of the electronic device 100 according to an embodiment may display, based on the control of the processor 101, various screens. The processor 101 may display, through the display 107, the two-dimensional image of the oral cavity of the subject 20, received from the three-dimensional scanner 200, and/or the three-dimensional image of the oral cavity, obtained by modeling the internal structure of the oral cavity in three-dimensions. For example, the two-dimensional image and/or the three-dimensional image of the oral cavity may be displayed through a specific application program. In the case above, the user 10 may edit, store, and delete the two-dimensional image and/or the three-dimensional image of the oral cavity.

The input device 109 of the electronic device 100 according to an embodiment may receive a command or data to be used for the element (e.g., the one or more processors 101) of the electronic device 100, from the outside (e.g., the user) of the electronic device 100. The input device 109 may include, for example, a microphone, a mouse, or a keyboard. According to an embodiment, the input device 109 may be coupled to the display 107 and implemented in the form of a touch sensor panel capable of recognizing the contact or proximity of various external objects.

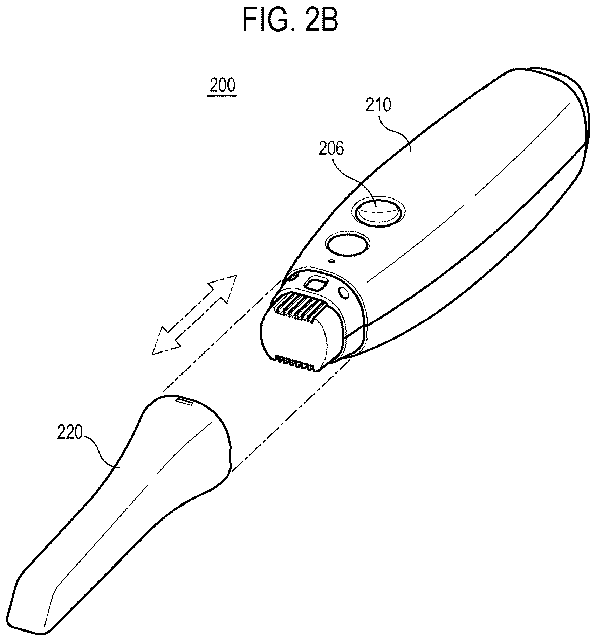

FIG. 2B is a perspective view of a three-dimensional scanner 200 according to an embodiment of the present disclosure. The three-dimensional scanner 200 according to an embodiment may include a main body 210 and a probe tip 220. The main body 210 of the three-dimensional scanner 200 may be formed in a shape allowing the user 10 to easily grip and use the same with hand. The probe tip 220 may be formed in a shape that is easy to be inserted into and withdrawn from the oral cavity of the subject 20. In addition, the main body 210 may be coupled to and separated from the probe tip 220. The elements of the three-dimensional scanner 200, described in FIG. 2A, may be arranged inside the main body 210. The one end of the main body 210 may have an opening that is opened so that light output from the light source 204 can be emitted to the subject 20. The light emitted through the opening may be reflected by the subject 20 and entered again through the opening. The reflected light entered through the opening may be captured by the camera 205 and may generate an image of the subject 20. The user 10 may start scanning by using the input device 206 (e.g., the button) of the three-dimensional scanner 200. For example, when the user 10 touches or presses the input device 206, the light from the light source 204 may be emitted to the subject 20.

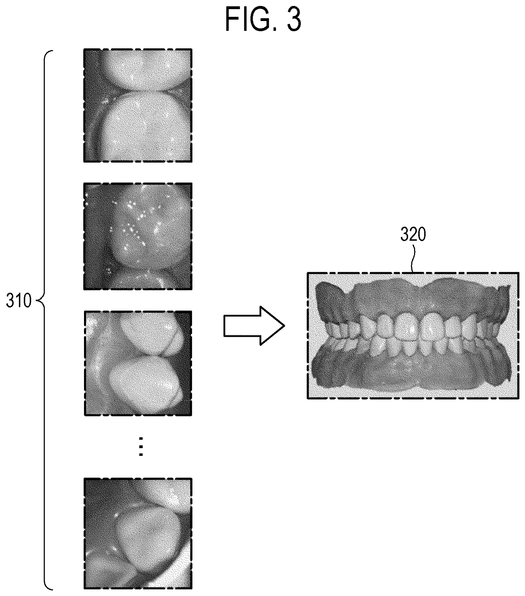

FIG. 3 illustrates a method of generating a three-dimensional image 320 of an oral cavity according to an embodiment of the present disclosure. The user 10 may scan the inside of the oral cavity of the subject 20 while moving the three-dimensional scanner 200, and in this case, the three-dimensional scanner 200 may obtain a plurality of two-dimensional image 310 of the oral cavity of the subject 20. For example, the three-dimensional scanner 200 may obtain a two-dimensional image of an area including an incisor of the subject 20, a two-dimensional image of an area including a posterior tooth of the subject 20, etc. The three-dimensional scanner 200 may transmit the plurality of obtained two-dimensional image 310 to the electronic device 100. According to another embodiment, the user 10 may scan a diagnostic model of the oral cavity while moving the three-dimensional scanner 200, and may obtain a plurality of two-dimensional image of the diagnostic model of the oral cavity. Hereinafter, for convenience of description, a case where an image of the oral cavity of the subject 20 is obtained by scanning the inside of the oral cavity of the subject 20 is described, but the present disclosure is not limited thereto.

The electronic device 100 according to an embodiment may convert each of the plurality of two-dimensional images 310 of the oral cavity of the subject 20 into a set of a plurality of points having three-dimensional coordinate values. For example, the electronic device 100 may convert each of the plurality of two-dimensional images 310 into a point cloud corresponding to a set of data points having three-dimensional coordinate values. For example, the point cloud set corresponding to three-dimensional coordinate values based on the plurality of two-dimensional images 310 may be stored as raw data of the oral cavity of the subject 20. The electronic device 100 may align the point clouds corresponds to the set of data points having the three-dimensional coordinate values, so as to complete the whole tooth model.

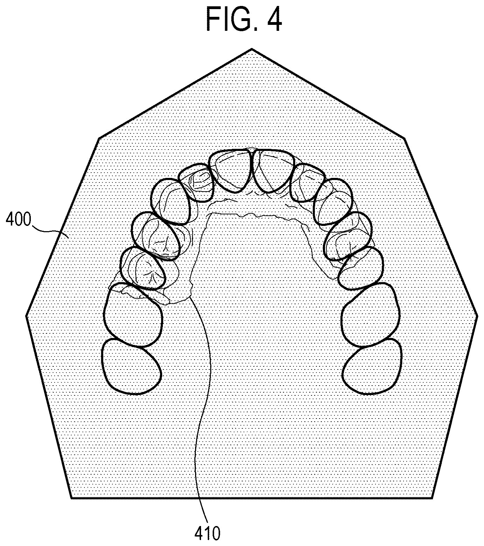

Hereinafter, the operation of aligning three-dimensional data of the oral cavity to the occlusal plane in FIGS. 4 to 13 may be performed by the processor 101 of the electronic device 100.

FIG. 4 illustrates aligning three-dimensional data of an oral cavity to an occlusal plane according to an embodiment of the present disclosure.

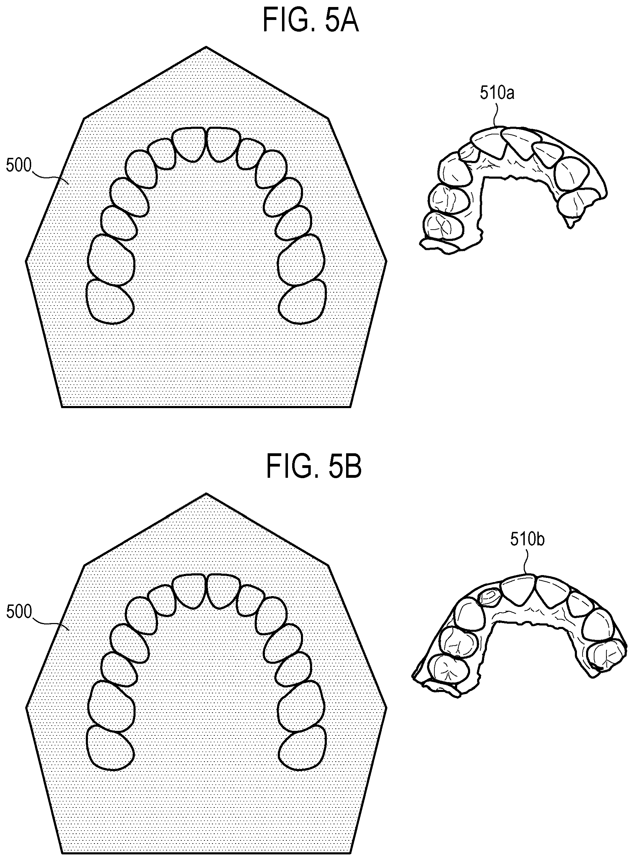

The processor may obtain first data 410 corresponding to three-dimensional data (e.g., the three-dimensional image in FIG. 1) of the oral cavity of a patient. The processor may align the first data 410 to an occlusal plane 400 so as to precisely perform subsequent processing for designing an oral appliance for prosthetic treatment of the patient. The occlusal plane 400 means a virtual plane configured based on the occlusion between the maxillary and mandibular dentitions in the oral cavity. For example, the processor may perform various processes for designing prostheses, crowns, bridges, implants, orthodontic appliances, and the like that are shaped to be suitable for the oral structure of the patient, by using the first data 410 aligned to the occlusal plane 400. Hereinafter, a method of aligning the first data 410 to the occlusal plane 400 is described in FIGS. 5A to 13 in detail.

FIG. 5A illustrates obtained first data according to an embodiment of the present disclosure.

FIG. 5B illustrates first data rotated based on a normal vector of an occlusal plane according to an embodiment of the present disclosure.

According to an embodiment, first data 510a or 510b may be data for a partial area of the oral cavity of a patient. According to an embodiment, the first data 510a or 510b may be data of an area into which a prosthesis is inserted in the oral cavity of the patient. For example, the first data 510a or 510b may be data of a mandibular arch (or data of a maxillary arch), or data of an area including some teeth among the mandibular arch data. According to an embodiment, the first data 510a or 510b may have a polygon file format. In this case, a surface indicated by the first data 510a or 510b may be represented in one or more triangles or quadrangles.

According to an embodiment, the processor may determine a normal vector indicated by the first data 510a or 510b. The processor may determine a normal vector indicated by the first data 510a or 510b, based on a normal vector of each of one or more polygons constituting the surface indicated by the first data 510a or 510b. For example, the processor may identify a tooth area of the oral cavity indicated by the first data 510a or 510b, determine a normal vector of each of one or more polygons in the tooth area, and determine a total of the respective normal vectors as a normal vector indicated by the first data 510a or 510b. According to another embodiment, the processor may determine a normal vector of a surface indicated by the first data 510a or 510b, based on a central line indicated by the first data 510a or 510b. The midline may mean a curve formed along an arch curve indicated by the first data 510a or 510b. The arch curve indicated by the first data 510a or 510b may be determined in a predetermined scheme, based on the position of each of the plurality of teeth indicated by the first data. The processor may define a plane including the curve formed through the arch curve, and determine a normal vector of the defined plane as a normal vector indicated by the first data 510a or 510b.

According to an embodiment, the first data 510a may be disposed on a three-dimensional space in a misaligned state with respect to an occlusal plane 500. The misaligned state indicates a state in which the normal vector of the occlusal plane 500 and the normal vector indicated by the first data 510a are not aligned. In this case, the occlusal plane 500 may be disposed to be parallel to a screen plane on which the occlusal plane 500 and the first data 510a are displayed, and the normal direction of the occlusal plane 500 and the normal direction of the screen plane may be aligned to each other. FIG. 5A illustrates first data 510a disposed on a three-dimensional space in a misaligned state with respect to the occlusal plane 500.

The processor may generate a rotation matrix for aligning the normal vector indicated by the first data 510a and the normal vector of the occlusal vector 500. The processor may calculate the normal vector of the occlusal plane 500, and generate, based on the normal vector indicated by the first data 510a and the normal vector of the occlusal vector 500, a rotation vector for rotating the first data 510a. The processor may rotate, based on the generated rotation matrix, the first data 510a so that the surface indicated by the first data 510a faces in the direction in which the occlusal plane 500 views. Referring to FIG. 5B, the processor may generate a rotation matrix for rotating the first data 510b so that the surface indicated by the obtained first data 510b faces in the direction in which the occlusal plane 500 views, and rotate the first data 510b so that the normal vector indicated by the first data 510b and the normal vector of the occlusal plane 500 are aligned to each other. Hereinafter, a state in which the normal vector indicated by the first data 510a or 510b and the normal vector of the occlusal plane 500 are aligned is assumed and described.

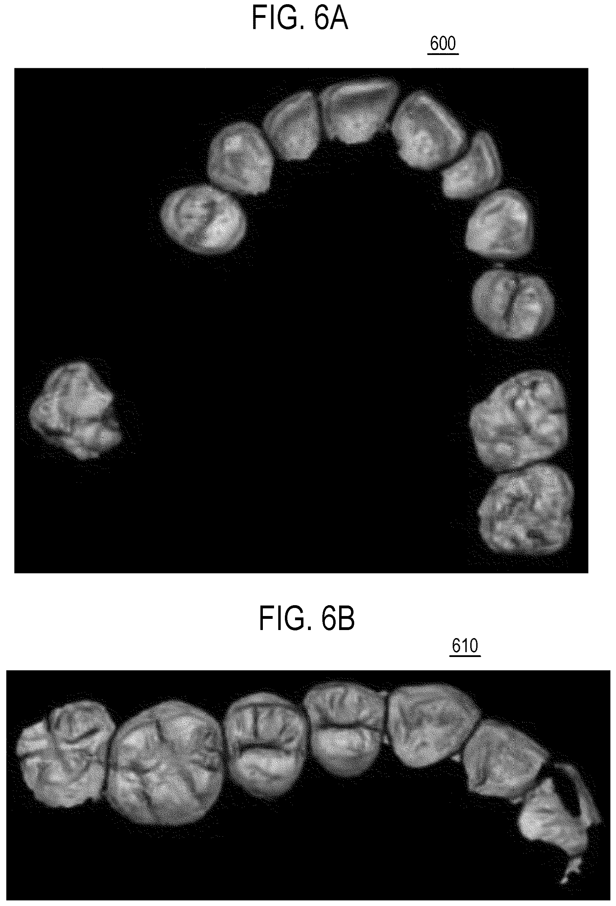

FIGS. 6A and 6B illustrate second data according to an embodiment of the present disclosure.

The processor may generate, based on first data corresponding to three-dimensional data, second data 600 or 610 corresponding to two-dimensional data of the oral cavity. According to an embodiment, the first data may include data of gingiva as well as data of teeth, and the processor may divide a tooth area and a gingiva area of the oral cavity indicated by the first data, and extract first data corresponding to the tooth area.

The processor may add, to the extracted first data of the tooth area, a color indicating the curvature of each point. The curvature of a tooth may mean the degree of bending of the tooth surface. According to an embodiment, the curvature of the tooth surface may be used for a training model to identify a tooth number of each tooth.

The processor may generate second data 600 or 610 by capturing a three-dimensional image indicated by the first data. For example, the processor may generate second data 600 or 610 indicating a two-dimensional image captured in the state in which the three-dimensional image of the tooth area, which is extracted from the first data and on which the curvature is indicated, is seen in the direction of the normal vector of the occlusal plane (i.e., the state of being exposed to a display screen). Referring to FIGS. 6A and 6B, the processor may generate second data 600 or 610 based on the first data. FIG. 6A may illustrate second data 600 for full arch data, and FIG. 6B may illustrate second data 610 for partial arch data. The full arch data means data indicating the entire arch of any one of the maxillary arch and the mandibular arch, and the partial arch data means data indicating only a part of the arch. As illustrated in FIGS. 6A and 6B, the second data 600 or 610 may further include a color indicating the curvature of each point. In the examples in FIGS. 6A and 6B, as the color of each tooth changes from a blue series to a yellow series and from a yellow series to a red series, the curvature at the corresponding point may have a relative larger magnitude.

According to an embodiment, the processor may determine the size of an image corresponding to the second data as a predetermined size. For example, the processor may determine the size of a two-dimensional image indicated by the second data as the size (e.g., 420 pixels in width and 380 pixels in height) of the two-dimensional image indicated by the input data of a training model to be described below.

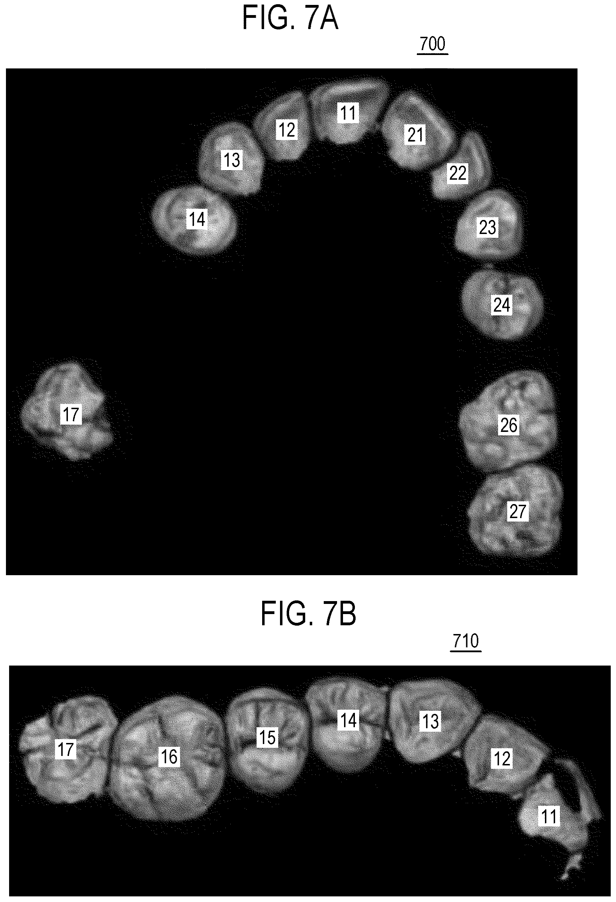

FIGS. 7A and 7B illustrate a result of inference of a training model for second data according to an embodiment of the present disclosure.

The processor may identify a plurality of teeth included in second data by using a training model, and determine two-dimensional center coordinates of each tooth. The training model may be an artificial neural network model, and may perform an inference process of receiving an input of the second data of the oral cavity, and identifying a tooth indicated by the second data. The processor may identify, based on an inference result 700 or 710 of the training model, each of the plurality of teeth indicated by the second data.

According to an embodiment, the processor may determine an identification number of each of the plurality of teeth indicated by the second data by using the training model. The identification number may be determined based on a predetermined system (e.g., the International Tooth Numbering System (Federation Dentaire Internationale (FDI)) or the Universal Numbering System) for classifying a plurality of teeth. For example, the training model may receive second data, and infer, based on at least one of the shapes of the plurality of teeth indicated by the second and the color indicating the curvature, numbers of the plurality of teeth indicated by the second data. The processor may identify the plurality of teeth indicated by the second data, based on the inference result 700 or 710 of the training model, including the identification number of each tooth.

According to an embodiment, the processor may determine two-dimensional center coordinates of each of the plurality of teeth indicated by the second data. The two-dimensional coordinates may mean the center of a tooth in the state indicated by the second data. The processor may determine, based on a bounding box, the two-dimensional center coordinates of each of the plurality of teeth. For example, the processor may generate a bounding box for each of the plurality of teeth. The bounding box means the smallest quadrangle including an area in which each of the plurality of teeth is positioned. The processor may determine, the center of each of the plurality of bounding boxes as two-dimensional center coordinates of a corresponding tooth. For example, the processor may determine an intersection point of two diagonals of the bounding box with reference to the bounding box. According to an embodiment, the processor may determine two-dimensional coordinates of each of the plurality of teeth by using the training model.

According to an embodiment, the training model may be trained using the plurality of two-dimensional images of the oral cavity as input data and data including tooth numbers and two-dimensional center coordinates of each of the plurality of teeth included in each image as label data.

FIG. 7A illustrates an inference result 700 obtained by inputting second data corresponding to FIG. 6A to a training model, and FIG. 7B illustrates an inference result 710 obtained by inputting second data corresponding to FIG. 6B to a training model. Hereinafter, the training model is described under the assumption that the identification number of the tooth is determined based on the International Tooth Numbering System (Federation Dentaire Internationale (FDI)), but it should not be construed that an embodiment in which the training model determines the identification number of a tooth is excluded.

Referring to FIG. 7A, the inference result 700 indicates identification numbers (17, 14, 13, 12, 11, 21, 22, 23, 24, 26, and 27 in order from the left) of each of the plurality of teeth indicated by the second data. Based on such an inference result 700, the processor may determine that the second data is full arch data of the maxillary arch. Referring to FIG. 7B, the inference result 710 may indicate identification numbers (17, 16, 15, 14, 13, 12, and 11 in order from the left) of each of the plurality of teeth indicated by the second data. According to a such inference result 710, the processor may determine that the second data is partial arch data of the right maxillary arch.

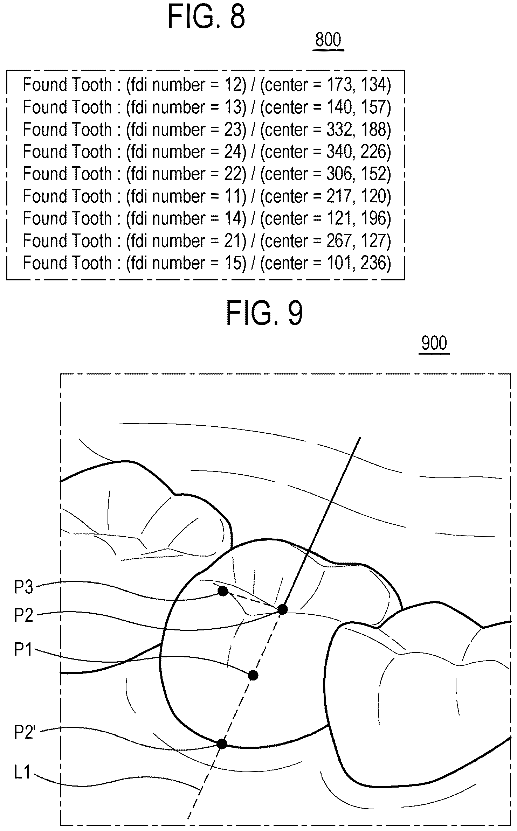

FIG. 8 illustrates an example of output data of a training model according to an embodiment of the present disclosure.

Referring to FIG. 8, a training model may output data 800 including identification numbers and two-dimensional center coordinates of each of the plurality of teeth included in the second data. For example, the output data 800 may indicate that, in the two-dimensional image indicated by the second data, the right maxillary central incisor (identification number 11), lateral incisor (identification number 12), canine (identification number 13), first premolar (identification number 14), and second premolar (identification number 15) are included, the left maxillary central incisor (identification number 21), lateral incisor (identification number 22), canine (identification number 23), and first premolar (identification number 24) are identified, the two-dimensional center coordinates of the right maxillary central incisor (identification number 11) are (217, 120), the two-dimensional center coordinates of the lateral incisor (identification number 12) are (173, 134), the two-dimensional center coordinates of the canine (identification number 13) are (140, 157), the two-dimensional center coordinates of the first premolar (identification number 14) are (121, 196), the two-dimensional center coordinates of the second premolar (identification number 15) are (101, 236), and the two-dimensional center coordinates of the left maxillary central incisor (identification number 21) are (267, 127), the two-dimensional center coordinates of the lateral incisor (identification number 22) are (306, 152), the two-dimensional center coordinates of the canine (identification number 23) are (332, 188), and the two-dimensional center coordinates of the first premolar (identification number 24) are (340, 226).

The processor may determine, based on the output data of the training model corresponding to the second data, the identification numbers and two-dimensional center coordinates of each of the plurality of teeth included in the second data.

FIG. 9 illustrates a process of determining three-dimensional center coordinates of each of a plurality of teeth by using a ray projection according to an embodiment of the present disclosure.

According to an embodiment, the processor may determine three-dimensional center coordinates corresponding two-dimensional center coordinates of each of the plurality of teeth. For example, the processor may determine a plurality of first points (P1) on a predetermined plane equal or parallel to the occlusal plane while corresponding to the two-dimensional center coordinates indicated by the two-dimensional images indicated by the second data. Next, the processor may determine, based on the plurality of first points (P1), a plurality of three-dimensional center coordinates according to a ray projection. For example, with respect to the plurality of first points (P1), the processor may define a projection line (Lp) which is in parallel to the normal line of the occlusal plane and passes the first point (P1), and determine an intersection point between the corresponding line (Lp) and a surface of each of the plurality of teeth as a plurality of second points (P2). When the intersection point between the projection line (Lp) and the surface of each tooth exists in the lower side (P2') and the upper side (P2), the processor may determine the occlusal-side intersection point located at the upper side as the second point (P2). The processor may determine the plurality of second points (P2) as three-dimensional center coordinates of each of the plurality of teeth.

According to another embodiment, the processor may determine a cusp point of each of the plurality of teeth as three-dimensional center coordinates of each of the plurality of teeth. For example, the processor may determine, as a third point (P3), a point at which the curvature has a maximum value among points obtained by moving the plurality of second points (P2) in the buccal direction and the lingual direction along the surface of each of the plurality of teeth. The processor may determine the third point (P3) as three-dimensional center coordinates of each of the plurality of teeth. The method for finding the cusp point of a tooth in the present disclosure is not limited by the description above, various methods of determining the cusp point regardless of the second point (P2) may be considered.

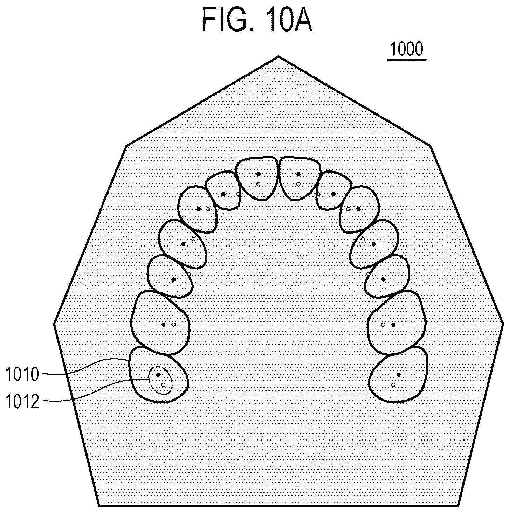

FIG. 10A illustrates reference center points on an occlusal plane according to an embodiment of the present disclosure.

The processor may align, based on three-dimensional center coordinates of each of a plurality of teeth indicated by first data and a reference center point corresponding to each of the plurality of teeth on an occlusal plane 1000, the first data to the occlusal plane 1000. According to an embodiment, the reference center points on the occlusal plane 1000 may be determined in the same scheme as a scheme of determining two-dimensional center coordinates of the plurality of teeth. Referring to FIGS. 10A, the processor may determine a reference center point 1012 for each of a maxillary tooth hand and a mandibular tooth that are in occlusion at a first position 1010 on the occlusal plane 1000. In FIG. 10A, a point (a hollow circle) positioned closer to the lingual side between two reference center points at each tooth position indicates a reference center point for the mandibular tooth, and the other point (a solid circle) indicates a reference center point for the maxillary tooth. The processor may determine, based on the reference center points corresponding to the plurality of teeth on the occlusal plane 1000, a transformation matrix for aligning the first data to the occlusal plane 1000.

According to an embodiment, the processor may determine one or more target teeth serving as a reference for determining the transformation matrix. The processor may classify the plurality of teeth indicated by the first data as one of a first tooth group and a second tooth group. For example, for each of the plurality of teeth indicated by the first data, when the corresponding tooth is an anterior tooth (e.g., a central incisor, a later incisor, or a canine), the processor may classify the same as the first tooth group, and when the corresponding tooth is a posterior teeth (e.g., a premolar or a molar), the processor may classify the same as the second tooth group. The sum of the number of teeth in the first tooth group and the number of teeth in the second tooth group may be identical to the number of the plurality of teeth indicated by the first data. The number of teeth in the first tooth group may be equal to or greater than 0. The number of teeth in the second tooth group may be equal to or greater than 0.

According to an embodiment, the processor may determine one or more target teeth based on whether a pair of teeth at positions corresponding to each other with reference to the midline of the oral cavity exists in each of the first tooth group and the second tooth group. According to an embodiment, the pair of teeth at positions corresponding to each other with reference to the midline may mean a pair of teeth which are positioned at different quadrants according to the FDI notation, and thus the tens digits of the tooth numbers are different, but the ones digits of the tooth numbers are identical. For example, the central incisor (tooth number 11) in quadrant 1 (on the right maxillary arch) and the central incisor (tooth number 21) in quadrant 2 (on the left maxillary arch) may be a pair of teeth at positions corresponding to each other, and the canine (tooth number 33) in quadrant 3 (on the left mandibular arch) and the canine (tooth number 43) in quadrant 4 (on the right mandibular arch) may be a pair of teeth at positions corresponding to each other.

According to an embodiment, the processor may determine whether a first tooth pair exists in the first tooth group, and may determine whether a second tooth pair exists in the second tooth group. When the first tooth pair and the second tooth pair exist, the processor may determine teeth (i.e., four teeth) included in the first tooth pair and the second tooth pair as target teeth. When the first tooth pair or the second tooth pair does not exist, the processor may determine all teeth indicated by the first data as target teeth.

According to an embodiment, when a plurality of tooth pairs at positions corresponding to each other exist in the first tooth group, the processor may determine, as a first tooth pair, a tooth pair including the closest tooth from the midline. For example, when the pairs of teeth at positions corresponding to each other in the first tooth group are (tooth number 11, tooth number 21), (tooth number 12, tooth number 22), and (tooth number 13, tooth number 23), the processor may determine the tooth pair (tooth number 11, tooth number 21) as the first tooth pair. The processor may determine a tooth pair including the closest tooth from the midline as the first tooth pair so as to more precisely align the central line when aligning the first data and the occlusal plane.

According to an embodiment, when a plurality of tooth pairs at positions corresponding to each other exist in the second group, the processor may determine two teeth as the second tooth pair according to a predetermined criterion. For example, the processor may set priorities for the posterior teeth, and may determine, as the second tooth pair, a pair of teeth including a tooth having the highest priority among the plurality of tooth pairs. For example, the processor may set the priorities in the order of (tooth number 16, tooth number 26), (tooth number 17, tooth number 27), (tooth number 18, tooth number 28), (tooth number 15, tooth number 25), and (tooth number 14, tooth number 24). For example, when all the tooth pairs of (tooth number 17, tooth number 27), (tooth number 18, tooth number 28), and (tooth number 14, tooth number 24) exist in the second tooth group, the processor may determine the tooth pair (tooth number 17, tooth number 27) having the highest priority among the tooth pairs as the second tooth pair. According to an embodiment, in general, the tooth pair (tooth number 16, tooth number 26), which is known to have the deepest roots among the teeth and thus shows the least movement during orthodontic treatment, may have the highest priority. The method in which the processor determines one of a plurality of tooth pairs at positions corresponding to each other in the second tooth group as the second tooth pair is not limited to the description above, and the processor may determine the second tooth pair in the same manner as determining the first tooth pair.

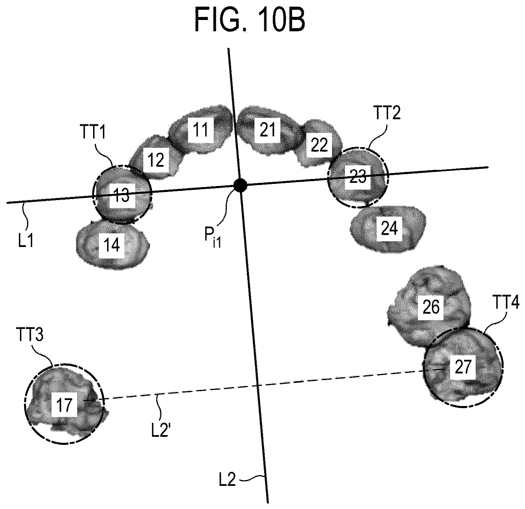

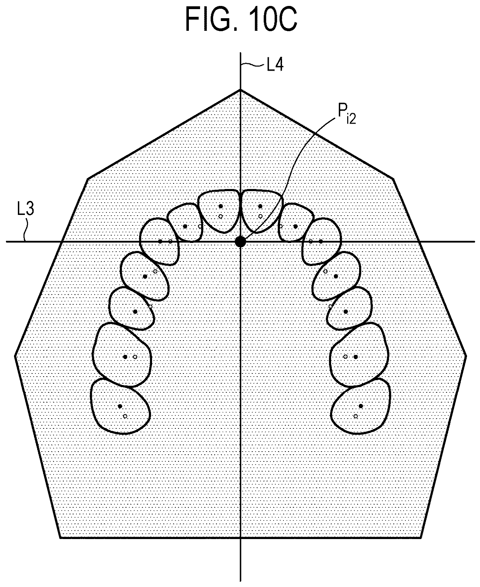

FIGS. 10B and 10C illustrate a process of aligning first data to an occlusal plane according to an embodiment of the present disclosure.

According to an embodiment, when both the first tooth pair and the second tooth pair exist, a predetermined scheme of aligning three-dimensional data indicating the oral cavity to an occlusal plane, based on two tooth pairs at positions corresponding to each other with reference to the midline of the oral cavity, may be used. For example, a transformation matrix may be generated so that two or more lines derived based on three-dimensional center points of four teeth of two tooth pairs and corresponding lines on the occlusal plane are aligned to each other. In a specific example, referring to FIGS. 10B and 10C, the processor may determine four teeth included in a first tooth pair (TT1, TT2) and a second tooth pair (TT3, TT4) as target teeth. The processor may determine, as a first straight line (L1), a straight line connecting three-dimensional center coordinates of each of the two teeth (TT1 and TT2) of the first tooth pair, and may determine, as a second straight line (L2), a perpendicular bisector of a line (L2') connecting three-dimensional center coordinates of each of the two teeth (TT3 and TT4) of the second tooth pair. Alternatively, the processor may obtain a cusp point based on the three-dimensional center coordinates for each of the teeth (TT1, TT2, TT3, and TT4), and then may determine the first straight line (L1) and the second straight line (L2) by using the cusp point rather than the three-dimensional center coordinates. The processor may determine a third straight line (L3) and a fourth straight line (L4) on the occlusal plane, which correspond to the first straight line (L1) and the second straight line (L2), respectively. The processor may generate a transformation matrix for aligning the first straight line (L1) and the third straight line (L3) and aligning the second straight line (L2) and the fourth straight line (L4). According to an embodiment, the processor may determine an intersection point (Pi1) of the first straight line (L1) and the second straight line (L2), and may align three-dimensional data indicated by the oral cavity to the occlusal plane by using the intersection point (Pi1). For example, the processor may determine a point (Pi2) on the occlusal plane (e.g., an intersection point of the third straight line (L3) and the fourth straight line (L4)), which corresponds to the intersection point (Pi1), and generate a transformation matrix of aligning the second straight line (L2) and the fourth straight line (L4) and aligning the intersection (Pi1) of the first straight line (L1) and the second straight line (L2) to the corresponding point (Pi2) on the occlusal plane.

When any one of the first tooth pair and the second tooth pair does not exist, the processor may generate a transformation matrix so that the three-dimensional center coordinates of each of the one or more target teeth (i.e., all of the plurality of teeth indicated by the first data) are moved as close as possible to a reference center point corresponding on the occlusal plane and the amount of movement of the oral cavity indicated by the first data can be minimized. For example, for each of the plurality of teeth indicated by the first data, the processor may determine a matrix for moving the three-dimensional center coordinates to the reference center point on the occlusal plane 1000 corresponding to each tooth. In a specific example, when the first data indicates five teeth, the processor may determine a first matrix for moving the three-dimensional center coordinates of the first tooth to the reference center point on the occlusal plane 1000 of the first tooth, determine a second matrix for moving the three-dimensional center coordinates of the second tooth to the reference center point on the occlusal plane 1000 of the second tooth, determine a third matrix for moving the three-dimensional center coordinates of the third tooth to the reference center point on the occlusal plane 1000 of the third tooth, determine a fourth matrix for moving the three-dimensional center coordinates of the fourth tooth to the reference center point on the occlusal plane 1000 of the fourth tooth, and determine a fifth matrix for moving the three-dimensional center coordinates of the fifth tooth to the reference center point on the occlusal plane 1000 of the fifth tooth. The processor may generate, based on the first matrix to the fifth matrix, a transformation matrix to minimize the amount of movement in the three-dimensional space of the oral cavity indicated by the first data. Accordingly, even when the first tooth pair and the second tooth pair do not exist, the first data can be aligned to the occlusal plane.

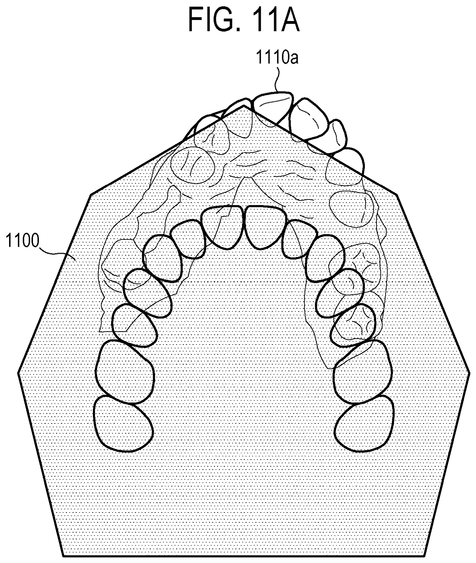

FIG. 11A illustrates full arch data before alignment to an occlusal plane according to an embodiment of the present disclosure.

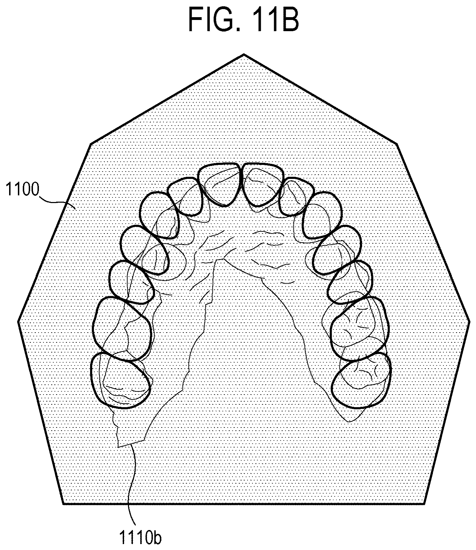

FIG. 11B illustrates full arch data obtained after alignment to an occlusal plane according to an embodiment of the present disclosure.

The processor may obtain first data 1110a corresponding to full arch data, and rotate, based on a rotation matrix, the first data 1110a in the direction of an occlusal plane 1100. The processor may generate, based on the first data 1110a, second data, and determine two-dimensional center coordinates and an identification number of each tooth by using the generated second data and a training model. The processor may determine, based on the two-dimensional center coordinates, three-dimensional center coordinates, and determine, based on whether a first tooth pair and a second tooth pair exist, one or more target teeth. Referring to FIG. 11A, both the first tooth pair and the second tooth pair exist in the full arch data, and thus the processor may generate, based on four teeth included in the first tooth pair and the second tooth pair, a transformation matrix. The processor may align, based on the transformation matrix, the first data 1110b to the occlusal plane 1100.

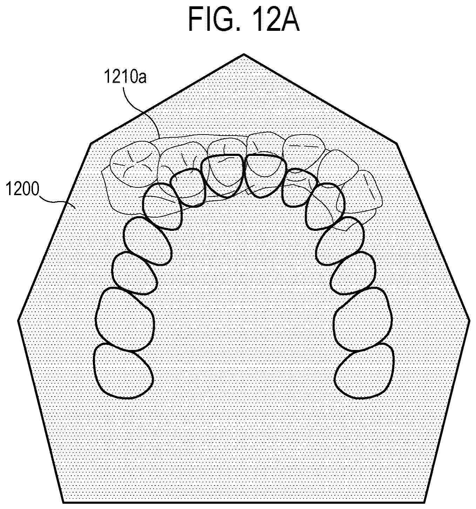

FIG. 12A illustrates partial arch data before alignment to an occlusal plane according to an embodiment of the present disclosure.

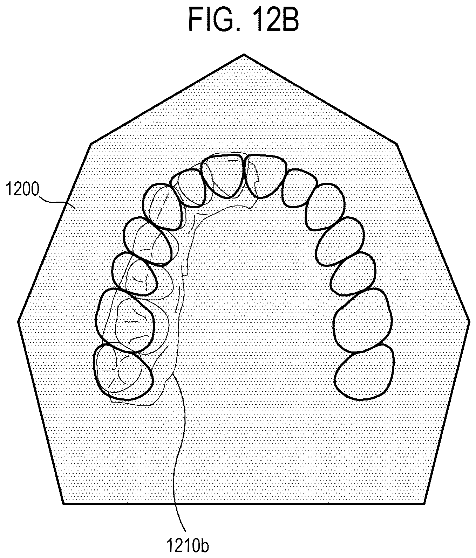

FIG. 12B illustrates partial arch data obtained after alignment to an occlusal plane according to an embodiment of the present disclosure.

A description redundant with the descriptions made in FIGS. 4 to 11B is omitted. The processor may obtain first data 1210a corresponding to partial arch data. There is no tooth pair in the partial arch data, and thus the processor may generate, based on all teeth included in the first data 1210a, a transformation matrix. The processor may align, based on the transformation matrix, the first data 1210b to an occlusal plane 1200. By using the method in the present disclosure, the first data can be precisely aligned to the occlusal plane even though there is no tooth pair at each corresponding positions with reference to a midline in the first data.

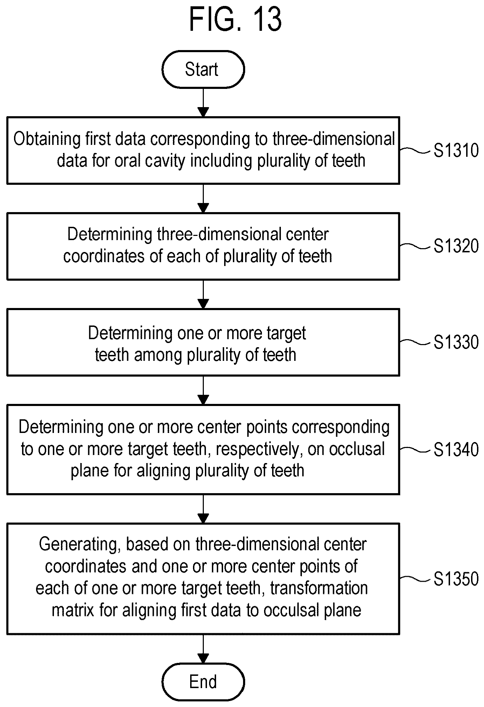

FIG. 13 is a flowchart illustrating a method of aligning three-dimensional data of an oral cavity to an occlusal plane according to an embodiment of the present disclosure.

An electronic device may obtain first data corresponding to three-dimensional data of an oral cavity including a plurality of teeth in operation S1310.

The electronic device may determine three-dimensional center coordinates of each of the plurality of teeth in operation S 1320.

The electronic device may determine one or more target teeth among the plurality of teeth in operation S1330.

The electronic device may determine one or more reference center points corresponding to the one or more target teeth, respectively, on the occlusal plane for aligning the plurality of teeth in operation S1340.

The electronic device may generate, based on the three-dimensional center coordinates and the one or more reference center points of each of the one or more target teeth, a transformation matrix for aligning the first data to the occlusal plane in operation S1350.

In the flowchart illustrated in FIG. 13, respective operations of the method or the algorithm according to the present disclosure are sequentially described, but the respective operations may also be performed according to an order of random combinations, in addition to the sequential performance. The description of according to the flowchart does not exclude changes or modifications in the method and/or the algorithm, and does not indicate that a random operation is necessary or preferable. In an embodiment, at least some operations may be performed in parallel, repeatedly, or heuristically. In an embodiment, at least some operations may be omitted or other operations may be added.

Various embodiments according to the present disclosure may be implemented as software in a machine-readable storage medium. The software may be software for implementing various embodiments disclosed in the present disclosure. The software may be inferred from various embodiments disclosed in the present disclosure by programmers in the technical field to which the present disclosure belongs. For example, the software may be a program including a machine-readable instruction (e.g., a code or a code segment). The device may be a device capable of operating according to an instruction loaded from the storage medium, and may be, for example, a computer. In an embodiment, the device may be an electronic device 100 according to embodiments of the present disclosure. In an embodiment, a processor of the device may execute the loaded instruction and allow elements of the device to perform a function corresponding to the instruction. In an embodiment, the processor may be a processor 101 according to embodiments of the present disclosure. The storage medium may be various types of recording medium storing machine-readable data. The storage medium may include a ROM, a RAM, a CD-ROM, a magnetic tape, a floppy disk, an optical data storage device, and the like. In an embodiment, the storage medium may be a memory 103. In an embodiment, the storage medium may be implemented in the form distributed to the computer systems which are connected through a network. The software may be distributed to and stored in computer systems and executed. The storage medium may be a non-transitory storage medium. The non-transitory storage medium means a tangible medium regardless of semipermanently or temporarily stored data, and does not include a transitorily transmitted signal.

Although the technical idea according to the present disclosure has been described by various embodiments, the technical idea according to the present disclosure may include various replacements, modifications, and changes that may be made within the scope of understanding by those skilled in the art. Furthermore, the replacements, modifications, and changes should be understood as being included in the scope of the accompanying claims.

## Claims

1. A method performed by an electronic device, the method comprising:
obtaining first data corresponding to three-dimensional data for an oral cavity comprising a plurality of teeth;
determining three-dimensional center coordinates of each of the plurality of teeth indicated by the first data;
determining one or more target teeth among the plurality of teeth;
determining one or more reference center points corresponding to the one or more target teeth, respectively, on an occlusal plane for aligning the plurality of teeth; and
generating a transformation matrix for aligning the first data to the occlusal plane, based on the three-dimensional center coordinates of each of the one or more target teeth and the one or more reference center points.

2. The method of Claim 1, wherein the determining of the one or more target teeth among the plurality of teeth comprises:
classifying each of the plurality of teeth as one of a first tooth group and a second tooth group;
determining whether there is a first pair of teeth at positions corresponding to each other with reference to a midline of the oral cavity and belonging to the first tooth group; and
determining whether there is a second pair of teeth at positions corresponding to each other with reference to the midline and belonging to the second tooth group.

3. The method of Claim 2, further comprising determining an identification number of each of the plurality of teeth,
wherein the determining of whether there is the first pair of teeth and the determining of whether there is the second pair of teeth are based on the identification number of each of the plurality of teeth.

4. The method of Claim 2, wherein the determining of the one or more target teeth among the plurality of teeth comprises, in response to determination that both the first pair of teeth and the second pair of teeth exist, determining four teeth included in the first pair of teeth and the second pair of teeth as the one or more target teeth.

5. The method of Claim 3, wherein the determining of the one or more target teeth among the plurality of teeth further comprises when there are a plurality of pairs of teeth at positions corresponding to each other with reference to the midline among one or more teeth included in the first tooth group, determining, as the first pair of teeth, a pair of teeth comprising a tooth closest from the midline among the plurality of teeth at positions corresponding to each other with reference to the midline.

6. The method of Claim 3, wherein the determining of the one or more target teeth among the plurality of teeth further comprises when there are a plurality of pairs of teeth at positions corresponding to each other with reference to the midline among one or more teeth included in the second tooth group, determining, as the second pair of teeth, a pair of teeth comprising a tooth closest from the midline among the plurality of teeth at positions corresponding to each other with reference to the midline.

7. The method of Claim 2, wherein the generating of the transformation matrix comprises, in response to determination that both the first pair of teeth and the second pair of teeth exist, generating the transformation matrix so that two or more straight lines derived based on three-dimensional center coordinates of the four teeth align with corresponding straight lines on the occlusal plane.

8. The method of Claim 2, wherein the determining of the one or more target teeth among the plurality of teeth comprises, in response to determination that at least one of the first pair of teeth and the second pair of teeth does not exist, determining all of the plurality of teeth as the one or more target teeth.

9. The method of Claim 8, wherein the generating of the transformation matrix comprises, in response to determination that at least one of the first pair of teeth and the second pair of teeth does not exist, generating the transformation matrix so that three-dimensional center coordinates of each of the one or more target teeth are moved as close as possible to a corresponding reference center point of the occlusal plane and an oral cavity movement amount indicated by the first data is minimized.

10. The method of Claim 1, wherein the determining of the three-dimensional center coordinates of each of the plurality of teeth indicated by the first data comprises:
generating, based on the first data, second data corresponding to two-dimensional data for the oral cavity;
identifying each of the plurality of teeth from the second data;
determining two-dimensional center coordinates of each of the plurality of teeth; and
determining three-dimensional center coordinates of each of the plurality of teeth, based on the two-dimensional center coordinates.

11. The method of Claim 10, wherein the generating of the second data corresponding to the two-dimensional data for the oral cavity comprises:
identifying a tooth area indicated by the first data, wherein the tooth area comprises the plurality of teeth;
adding a color indicating a curvature to each point of the tooth area; and
generating the second data, based on data of the tooth area in which the color is added, among the first data.

12. The method of Claim 11, wherein the determining of the two-dimensional center coordinates of each of the plurality of teeth comprises:
identifying each of the plurality of teeth indicated by the second data;
generating a plurality of bounding boxes corresponding to the plurality of teeth, respectively, wherein the plurality of boxes have a smallest quadrilateral shape comprising an area in which each of the plurality of teeth is positioned in the second data; and
determining a center of each of the plurality of bounding boxes as two-dimensional center coordinates of a corresponding tooth.

13. The method of Claim 10, wherein the determining of the two-dimensional center coordinates of each of the plurality of teeth comprises applying the second data to a training model to obtain output data, wherein the output data identifies each of the plurality of teeth and indicates the two-dimensional center coordinates of each of the plurality of teeth.

14. The method of Claim 10, wherein the determining of the three-dimensional center coordinates of each of the plurality of teeth comprises:
determining a plurality of first points which correspond to the two-dimensional center coordinates of each of the plurality of teeth and are on a plane identical or parallel to the occlusal plane;
determining a plurality of second points on surfaces of the plurality of teeth, which correspond to the plurality of first points, wherein each of the plurality of second points is an intersection point between a straight line which is parallel to a normal line of the occlusal plane and passes through a corresponding first point and a surface of each of the plurality of teeth; and
determining the plurality of second points as the three-dimensional center coordinates of each of the plurality of teeth.

15. The method of Claim 14, wherein the determining of the three-dimensional center coordinates of each of the plurality of teeth comprises:
determining, based on the plurality of second points, a plurality of third points corresponding to cusp points of each of the plurality of teeth; and
determining the plurality of third points as the three-dimensional center coordinates of each of the plurality of teeth.

16. The method of Claim 15, wherein the determining of the third point of each of the plurality of teeth comprises determining, as the third point of each of the plurality of teeth, a point at which a curvature has a maximum value among points obtained by moving the plurality of second points in a buccal direction and a lingual direction along the surface of each of the plurality of teeth.

17. The method of Claim 1, further comprising aligning the first data to the occlusal plane, based on the transformation matrix.

18. The method of Claim 1, further comprising:
segmenting surfaces of a plurality of teeth indicated by the first data into one or more polygons;
determining one or more first normal vectors for each of the one or more polygons;
determining a second normal vector corresponding to the first data, based on the one or more first normal vectors;
determining a third normal vector corresponding to a normal vector of the occlusal plane; and
generating a rotation matrix for rotating the first data so that the second normal vector aligns with the third normal vector.

19. An electronic device comprising:
one or more processors; and
one or more memories in which instructions executed by the one or more processes are stored,
wherein the one or more processors are configured to, when the instructions are executed by the one or more processors, execute the method according to any one of Claims 1 to 18.

20. A non-transitory computer-readable recording medium recording instructions which, when executed by one or more processors, cause the one or more processors to perform operations,
wherein the instructions are configured to cause the one or more processors to execute the method according to any one of Claims 1 to 18.
